Europäisches Patentamt

European Patent Office

Office européen des brevets

(19)

(11) Veröffentlichungsnummer : **0 084 093**
**B1**

(12) # EUROPÄISCHE PATENTSCHRIFT

(45) Veröffentlichungstag der Patentschrift :
30.01.85

(21) Anmeldenummer : 82110420.5

(22) Anmeldetag : 11.11.82

(51) Int. Cl.⁴ : **A 61 G 13/00**

(54) **Patientenliege.**

(30) Priorität : 15.01.82 DE 3201021

(43) Veröffentlichungstag der Anmeldung :
27.07.83 Patentblatt 83/30

(45) Bekanntmachung des Hinweises auf die Patenterteilung : 30.01.85 Patentblatt 85/05

(84) Benannte Vertragsstaaten :
**CH DE FR GB IT LI NL SE**

(56) Entgegenhaltungen :
BE-A-   788 456
DE-C-   882 476
FR-A- 2 247 195

(73) Patentinhaber : DORNIER SYSTEM GmbH
Postfach 1360
D-7990 Friedrichshafen (DE)

(72) Erfinder : Forssmann, Bernd, Dr. rer.nat.
Salemweg 6
D-7990 Friedrichshafen 1 (DE)
Erfinder : Gerth, Hans-Heinrich, Dr.-Ing.
Hans-Dieter-Strasse 26
D-7758 Meersburg (DE)
Erfinder : Zech, Hendrik, Ing. grad.
Rauensteinstrasse 80
D-7770 Überlingen (DE)
Erfinder : Chaussy, Christian, Prof. Dr. med.
Friedenstrasse 13a
D-8034 Germering (DE)

(74) Vertreter : Landsmann, Ralf, Dipl.-Ing.
Kleeweg 3
D-7990 Friedrichshafen 1 (DE)

## Beschreibung

Die Erfindung betrifft eine Liege zur sicheren, druckstellenfreien, nicht überdehnenden Lagerung und Fixierung eines voll- oder teilnarkotisierten Patienten z. B. zur berührungsfreien Zerkleinerung von Nierensteinen mittels Stoßwellen.

Dabei werden durch Zündung einer Unterwasserfunkenstrecke Stoßwellen erzeugt, mittels eines Reflektors fokussiert und in den Körper des Patienten geleitet, wo sie den Stein zerkleinern (DE-C-23 51 247). Um die Stoßwellen verletzungsfrei ein- und auskoppeln zu können, wird der Körper des Patienten in einer mit entgastem Wasser gefüllten Wanne gelagert. Der Nierenstein wird mit Röntgenstrahlen geortet. Der Patent muss exakt in die Position gebracht werden, in welcher der Stein genau im Brennpunkt des ortsfesten Stoßwellenreflektors liegt und dort über längere Zeit gehalten werden. Bei einer Stoßwellenbehandlung ist der Patient voll- oder teilnarkotisiert.

Aufgabe der Erfindung ist es, eine Liege zu schaffen, mit der ein voll- oder teilnarkotisierter Patient so in einer Wanne positioniert werden kann, dass ein bestimmter Punkt in seinem Körper z. B. ein Nierenstein in einen festen Raumpunkt (2. Brennpunkt des Ellipsoids) über längere Zeit fixiert werden kann. Dabei müssen Druckstellen, an denen der Blutkreislauf blockiert wird, vermieden werden. Wegen der langen Liegezeit könnten diese zum teilweisen Absterben von Körperteilen führen. Auch muss in der Narkose eine Überdehnung von Körperpartien verhindert werden. Denn schon kurzfristige Überdehnungen, wie sie z. B. bei Sportverletzungen auftreten, können schmerzhaft sein. Das gilt umso mehr für längere Überlastungen.

Die Aufgabe wird gelöst mit einer Liege, die die in Anspruch 1 genannten Merkmale besitzt. Ausbildungen der Erfindung sind Gegenstände von Unteransprüchen.

Der Erfindung liegt die Erkenntnis zugrunde, dass ein menschlicher Körper nur an der Schulter und an den Beinen unterstützt werden muss, um eine sichere Lage zu erreichen. Überraschenderweise wurde gefunden, dass der narkotisierte, schlaffe Körper sowohl in der Luft, als auch im Wasser nicht durch das « Loch » zwischen Schulter und Beinauflagen sackt. Dies wird erreicht durch eine starke Gegeneinanderstellung von Schulterstützen, Oberschenkelschalen und Unterschenkelschalen. Der Körper wird in eine doppelte Prismenlage gebracht. Rumpf und Oberschenkel ruhen in einer V-förmigen « Rinne », Oberschenkel und Unterschenkel ruhen auf einer « Prismenführung ».

Die für die Behandlung angestrebte Hohlkreuzlage wird nicht — wie man erwarten könnte — durch eine erhöhte Unterstützung im Hüftbereich erreciht, sondern überraschenderweise reicht die erfindungsgemässe Schulterauflage und Armfixierung neben dem Kopf aus, wobei der gesamte Rückenbereich unterhalb der Schulter bis zum Gesäß frei zugänglich und nicht unterstützt bleibt.

Die Erfindung bietet folgende Vorteile :
— Geeignet für alle normalen Grössen erwachsener Patienten
— Freie Zugänglichkeit des Rumpfbereiches, insbesondere der Nierengegend
— Feste Positionierung und Fixierung des Patienten ohne Druckstellen und Bänderüberdehnung
— Auch für vollnarkotisierte Patienten geeignet
— Einfacher Umbau für die Behandlung der rechten oder der linken Niere.

Patientenliegen mit den im Anspruch 1 genannten Merkmalen sind in vielerlei Ausführungen realisierbar. Bei Verwendung einer Wanne ist eine Befestigung der Auflageflächen an Bügeln von oben vorteilhaft. Auch in den Befestigungen und Führungen der Auflageflächen hat der Konstrukteur viele Möglichkeiten die erfindungsgemässe Lehre auszuführen.

Eine vorteilhafte Ausführungsform einer erfindungsgemässen Liege wird anhand der folgenden Figuren näher erläutert.

Es zeigen :
Figur 1   eine Liege von der Seite,
Figur 2   ein Detail,
Figur 3   die Liege der Fig. 1 von oben.

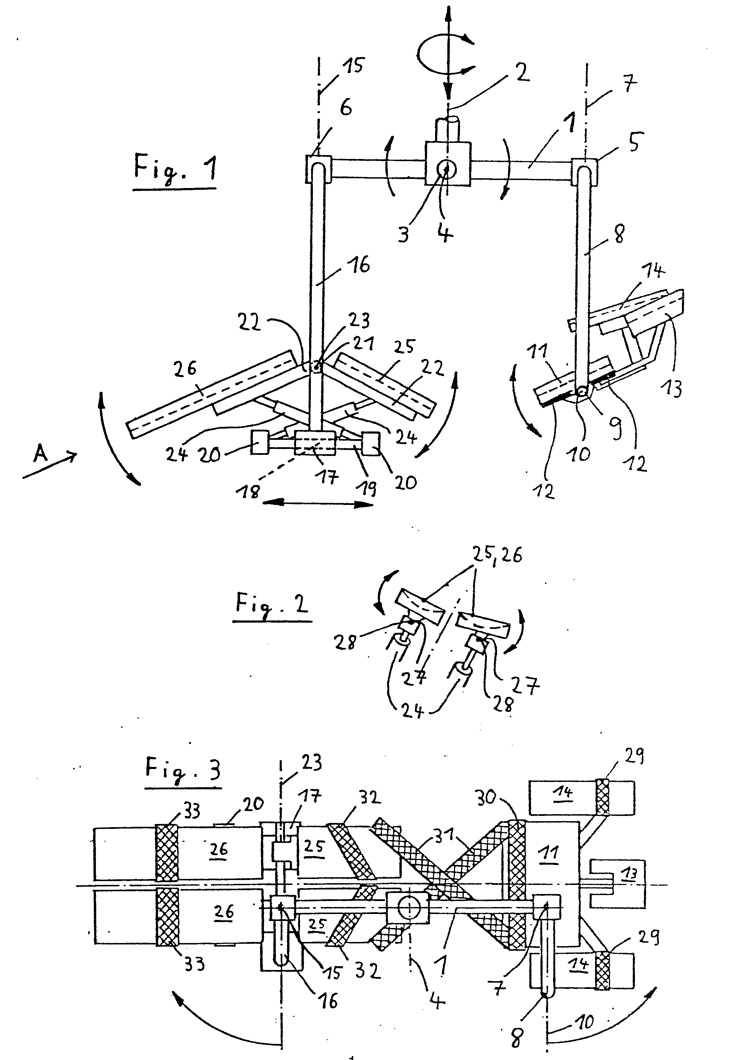

Fig. 1 zeigt eine Seitenansicht einer erfindungsgemässen Liege. An einem hier nicht gezeigten Kran befindet sich ein Gabelgelenk 3, welches um die Achse 2 schwenkbar und entlang der Achse 2 hebbar ist, wie es die Pfeile andeuten. Am Gabelgelenk 3 ist ein in seinem Mittelbereich gelagerter und um die Achse 4 schwenk- und arretierbarer Tragbalken 1 befestigt, der in den beiden Gelenken 5 und 6 endet. Die beiden Gelenke 5 und 6 besitzen zwei um 180° versetzte diskrete Stellungen. Am Gelenk 5 ist mittels eines um die Achse 7 drehbaren, im wesentlichen U-förmigen Tragbügels 8 eine im Gelenk 9 um die Achse 10 drehbare und arretierbare Schulterauflage 11 befestigt. Die Schulterauflage 11 ist symmetrisch aufgebaut und besitzt an ihrer Unterseite links und rechts der Drehachse 10 je drei Führungen 12. In die Führungen einer Seite (in Fig. 1 rechts) werden eine Kopfstütze 13 und zwei Armauflagen 14 so eingesteckt und arretiert, dass die Unterarme neben dem Kopf (hands-up-Stellung) liegen.

Am Gelenk 6 ist mittels eines um die Achse 15 drehbaren, im wesentlichen L-förmigen, zweiten Tragbügels 16 eine Quertraverse 17 befestigt, die zwei parallele, längsverlaufende Führungen 18 enthält. In den Führungen liegen zwei längsverschiebbare und arretierbare Schienen 19, die das Fußteil tragen. Die Schienen 19 sind mittels zweier Querträger 20 verbunden. Oberhalb der Schienenmitte befinden sich die Gelenke 21, an denen vier Schalenträger 22 um die Achse 23 drehbar befestigt sind. Die Neigung der Schalenträger 22 wird durch die Länge der Spindeln 24 bestimmt, die kreuzweise jeweils zwischen einem Querträger 20 und dem gegenüberliegenden Schalenträger 22 liegen. Auf jedem Schalenträger 22 wird eine Oberschenkelschale 25 oder eine Unterschenkelschale 26 befestigt.

Fig. 2 zeigt zwei Schalen 25 oder 26 aus der Blickrichtung A (Fig. 1). Die Schale 26 kann ebenso wie die Schale 25 in den Gelenken 27 um die Achse 28 verkippt werden (rechtes Teilbild) oder in Neutralstellung — unverkippt — arretiert werden (linkes Teilbild). Die rechts gezeigte Schale ist zusätzlich noch um einen gewissen Betrag angehoben.

Fig. 3 zeigt die in Fig. 1 gezeigte Liege von oben. Zusätzlich zu den in der Figurenbeschreibung zu Fig. 1 genannten Bauteilen ist hier ein Gurtsystem 29 bis 33 zur Fixierung des Körpers und zur Aufnahme der Auftriebskraft in der wassergefüllten Wanne gezeigt. Die Anordnung der Armauflagen 14 neben dem Kopf führt zu einer Hohlkreuzlage des Patienten. Durch diese Lage wird die Röntgenortung des Nierensteins erleichtert.

Zu erkennen ist die aussermittige Anordnung des Trägers 1 gegenüber der Längsachse der Liege. Diese asymmetrische Aufhängung dient der besseren Zugänglichkeit der Niere für Ortung und Behandlung in der Wanne. Die in den Fig. 1 und 3 gezeigte Liege ist für eine Behandlung der rechten Niere aufgebaut. Ein Umbau auf Behandlung der linken Niere ist möglich und schnell durchführbar. Dazu werden die Gurte 31 gelöst, die Auflageflächen für Kopf 13, Arme 14, Beine 25, 26 abgenommen, der Tragbügel 8 und mit ihm die Schulterstütze 11 um 180° um die Achse 7 gedreht (Pfeil in Fig. 3), der Tragbügel 16 und mit ihm das Fußteil um 180° um die Achse 15 gedreht (Pfeil in Fig. 3) und die abgenommenen Bauteile 13, 14, 25, 26 wieder angesteckt.

Zur Ortung des Nierensteins und zu dessen Zerkleinerung muss der Patient eine leichte Seitenlage einnehmen. Diese wird durch einseitiges Anheben einer Beinschale 25 erreicht. Durch die daraufhin erfolgende Verdrehung des Körpers verkippen alle vier Beinschalen 25, 26, was zu einer sehr günstigen Lage der Beine führt. Durch das selbsttätige Verkippen der Schalen werden dabei Druckstellen oder ein Blutstau in den narkotisierten Körperteilen vermieden.

Durch ihre vielfältigen Verstellmöglichkeiten (Pfeile im Bild 1) kann die Liege exakt auf die Grösse und den Körperbau des jeweiligen Patienten eingestellt werden. Dies geschieht z. B. durch ein Probeliegen im nichtnarkotisierten Zustand und einem dabei erfolgten Aufschreiben der Spindel- und Gelenkstellungen zur Reproduktion der optimalen Stellung am Behandlungstag. So wird erreicht, dass Druckstellen vermieden werden und keine Bänderüberdehnungen auftreten.

Die Liege kann auch zur anatomisch richtigen Lagerung oder Fixierung von Wirbelsäulenverletzten oder von Querschnittsgelähmten verwendet werden.

**Ansprüche**

1. Liege zur sicheren, druckstellenfreien, nicht überdehnenden Lagerung und Fixierung eines voll- oder teilnarkotisierten Patienten z. B. zur berührungsfreien Nierensteinzerkleinerung mittels Stoßwellen, dadurch gekennzeichnet, dass der Patient durch stark gegeneinander neigbare und einzeln verstellbare Auflageflächen (11, 25, 26) im Schulterbereich und an den Beinen in eine Rückenlage mit leicht angehobenem Oberkörper und angewinkelten Beinen mit freiem Rücken und Rektalbereich und durch die Lagerung (14) der Unterarme neben dem Kopf in eine stabile Hohlkreuzlage gebracht wird.

2. Patientenliege nach Anspruch 1, dadurch gekennzeichnet,

a) dass ein Tragbalken (1) in seinem Mittelbereich an einem Gelenk (3) um eine Achse (4) schwenkbar und arretierbar gelagert ist und in zwei Gelenken (5, 6) endet, die je zwei um 180° versetzte diskrete Stellungen haben,

b) dass in dem einen unter a) genannten Gelenk (5) mittels eines um eine Achse (7) schwenkbaren, im wesentlichen U-förmigen Tragbügels (8) eine in einem Gelenk (9) um eine Achse (10) schwenkbare und arretierbare Schulterauflage (11) befestigt ist,

c) dass an der Unterseite der Schulterauflage (11) zu beiden Seiten der Drehachse (10) Führungen (12) zum Einstecken und Arretieren je einer Kopfstütze (13) und je zweier Armauflagen (14) vorgesehen sind, wobei die Armauflagen (14) so angeordnet sind, dass die Unterarme neben dem Kopf liegen,

d) dass in dem zweiten unter a) genannten Gelenk (6) mittels eines um eine Achse (15) schwenkbaren, im wesentlichen L-förmigen zweiten Tragbügel (16) eine Quertraverse (17) befestigt ist, die zwei parallel ausgerichtete, in Längsrichtung der Liege verlaufende Führungen (18) enthält,

e) dass ein Fußteil vorhanden ist, welches auf zwei Schienen (19), die in den Führungen (18) der Quertraverse (17) längsverschiebbar angeordnet sind, ruht und vier mittels Gelenken (21) um eine Achse (23) schwenkbare Schalenträger (22) besitzt, deren Neigung durch die Längenänderung von Spindeln (24) einstellbar ist, wobei die Spindeln (24) kreuzweise zwischen den Schalenträgern (22) und zwei Querträgern (20), die an den Schienenenden befestigt sind, angeordnet sind,

f) dass an jedem Schalenträger (22) eine Oberschenkelschale (25) oder eine Unterschenkelschale (26) befestigt ist.

3. Patientenliege nach Anspruch 1 oder 2, dadurch gekennzeichnet, dass jede Schenkelschale (25, 26) um eine zu ihrer Längsachse parallele Achse (28) in beiden Richtungen um einen gewissen Betrag kippbar und in der Neutralstellung arretierbar ist.

4. Patientenliege nach einem der vorhergehenden Ansprüche, dadurch gekennzeichnet, dass an den Arm- und Beinauflagen (14, 25, 26) Gurte (29, 32, 33) zum Fixieren der jeweiligen Extremität vorgesehen sind und daß ferner ein Gurtsystem (30, 31) vorgesehen ist, welches den Oberkörper und den Rumpf zwischen den Auflageflächen für die Oberschenkel (25) und die Schulterpartie (11) fixiert.

5. Patientenliege nach einem der vorhergehenden Ansprüche, dadurch gekennzeichnet, dass die Auflageflächen (11, 13, 14, 25, 26) mit wasserdichtem, geschlossenporigem Schaumstoff gepolstert sind.

6. Patientenliege nach einem der vorhergehenden Ansprüche, dadurch gekennzeichnet, dass sich der Tragbalken (1) und damit der Aufhängepunkt neben der Symmetrieachse der Liege befindet.

## Claims

1. A couch for reliably supporting and fixing in place a completely or partially anesthesized patient in a strain-free and compression-free manner, for instance to comminute renal calculi in a contact-less manner by means of shock waves, characterized by inclined and individually adjustable support surfaces (11, 25, 26) in the areas of the shoulder and the legs whereby the trunk is slightly raised, the legs are angled, the back and the rectal area are free, and the lower arms are supported (14) near the head, the patient thereby being in a stable, hollow-back position.

2. A couch for patients according to claim 1 characterized by
a) a support beam (1) supported at the center thereof in an articulation (3) so as to be pivotal about an axis (4) and lockable in place and terminating in two hinges (5, 6) each assuming two positions offset by 180°,
b) a shoulder support (11) pivotal about an axis (10) in an articulation (9) and lockable in place, secured to one of the hinges (5) cited in (a) by an essentially U-shaped support post (8) pivotal about an axis (7),
c) guides (12) at the underside of the shoulder support (11) on both sides of the axis of rotation (10) to insert and lock in place in each case on head rest (13) and two arm rests (14), being so arranged that the lower arms lie next to the head,
d) a cross-bar (17), containing two parallel guides (18) extending in the longitudinal direction of the couch, mounted by means of a second, essentially L-shaped support post (16) pivotal about an axis (15) in the second hinge (6) cited in (a),
e) a foot portion resting on two rails (19) mounted in a longitudinally displaceable manner in the guides (18) of the cross-bar (17) and including four pan supports (22) pivotal by means of four articulations (21) about an axis (23), the inclination of said pan supports being adjustable by changing the lengths of spindles (24), the spindles (24) being arranged crosswise between the pan supports (22) and two cross-beams (20) secured to the rail ends,

f) and a thigh pan (25) or a lower leg pan (26) mounted to each pan support (22).

3. A couch for patients according to claim 1 or 2, characterized in that each thigh pan or lower leg pan (25, 26) can be tipped by a given amount in both directions about an axis (28) parallel to the longitudinal axis of said thigh pan or lower leg pan and can be locked into its neutral position.

4. A couch for patients according to one of the preceding claims, characterized by straps (29, 32, 33), for securing a particular extremity, at the arm and leg rests (14, 25, 26) and by a strap system (30, 31) for securing the upper body and the trunk between the support surfaces for the thighs (25) and the shoulder (11).

5. A couch for patients according to one of the preceding claims, characterized in that the rest surfaces (11, 13, 14, 25, 26) are padded with a watertight, closed-pore foam.

6. A couch for patients according to one of the preceding claims, characterized in that the support beam (1), and hence the suspension point thereof, is offset from the axis of symmetry of the couch.

## Revendications

1. Chaise-lit pour un positionnement et une fixation sûre, exempts d'endroits de pression et sans allongement excessif d'un patient totalement ou partiellement anesthésié, par exemple pour une fragmentation sans contact de calculs rénaux au moyen d'ondes de choc, caractérisée par le fait que le patient est amené dans une position dorsale avec une partie supérieure du corps légèrement soulevée et des jambes repliées avec le dos et la région rectale libres, au moyen de surfaces d'appui (11, 25, 26) fortement inclinables l'une par rapport à l'autre et réglables isolément dans la région des épaules et aux jambes, et dans une position en croix concave stable par l'appui (14) des avant-bras au voisinage de la tête.

2. Chaise-lit pour patient selon la revendication 1, caractérisée par le fait
a) qu'une poutre porteuse (1) est montée dans sa zone médiane de manière pivotante autour d'un axe (4) et blocable sur une articulation (3) et se termine par deux articulations (5, 6) qui possèdent chacune deux positions séparées décalées de 180°,
b) que dans l'une des articulations (5) mentionnées sous a), un appui d'épaule (11), pivotant dans une articulation (9) autour d'un axe (10) et blocable, est fixé dans un étrier porteur (8) sensiblement en forme de U pivotant autour d'un axe (7),
c) que sur le côté inférieur de l'appui d'épaule (11) des deux côtés de l'axe de rotation (10) sont prévus des guides (12) pour la mise en place et le blocage d'un appui de tête (13) et de deux appuis de bras (14), les appuis de bras (14) étant disposés de manière que les avant-bras se trouvent placés près de la tête,

d) que dans la deuxième articulation (6) mentionnée sous a) est fixée, au moyen d'un second étrier porteur (16) sensiblement en forme de L et pivotant autour d'un axe (15), une traverse transversale (17) qui contient deux guides parallèles s'étendant dans le sens longitudinal de la chaise-lit,

e) qu'il est prévu une partie de pied qui repose sur deux rails (19) disposés de façon déplaçable longitudinalement dans les guides (18) de la traverse transversale (17) et possède quatre porte-coquilles (22) pivotant au moyen d'articulations (21) autour d'un axe (23) et dont l'inclinaison est réglable par modification de longueurs de broches (24), les broches (24) étant disposées en croix entre les porte-coquilles (22) et deux supports transversaux (20) fixés aux extrémités des rails,

f) qu'à chaque porte-coquille est fixée une coquille de cuisse (25) ou une coquille de jambe (26).

3. Chaise-lit pour patient selon la revendication 1 ou 2, caractérisée par le fait que chaque coquille (25, 26) est susceptible d'être basculée autour d'un axe (28) parallèle à son axe longitudinal dans les deux sens d'une certaine valeur et est blocable dans la position neutre.

4. Chaise-lit pour patient selon l'une des revendications précédentes, caractérisée par le fait que sur les appuis de bras et de jambes (14, 25, 26) sont prévues des ceintures (29, 32, 33) pour la fixation de l'extrémité correspondante et qu'en outre un système de ceintures (30, 31) est prévu pour fixer la partie supérieure du corps et le tronc entre les surfaces d'appui de cuisse (25) et d'épaule (11).

5. Chaise-lit pour patient selon l'une des revendications précédentes, caractérisée par le fait que les surfaces d'appui (11, 13, 14, 25, 26) sont rembourrées d'une mousse étanche à l'eau à pores fermés.

6. Chaise-lit pour patient selon l'une des revendications précédentes, caractérisée par le fait que la poutre porteuse (13) et ainsi le point de suspension se trouvent près de l'axe de symétrie de la chaise-lit.

Fig. 1

Fig. 2

Fig. 3